# EUROPEAN PATENT SPECIFICATION

(11) **EP 3 845 232 B1**
(45) Date of publication and mention of the grant of the patent: **09.10.2024**
(21) Application number: 19854229.2
(22) Date of filing: 29.08.2019
(51) Int. Cl.: A61K 31/575, A61K 8/63, A61P 3/04, A61Q 19/00, A61K 9/00, A61K 47/10, A61Q 19/06

(54) **TRANSDERMAL USE OF PHYTOSTENONE FOR REDUCING SUBCUTANEOUS FAT**
TRANSDERMALE VERWENDUNG VON PHYTOSTENON ZUR REDUZIERUNG VON SUBKUTANEM FETT
UTILISATION TRANSDERMIQUE DE PHYTOSTÉNONE POUR RÉDUIRE LA GRAISSE SOUS-CUTANÉE

(30) Priority: 30.08.2018 JP 2018161943
(43) Date of publication of application: 07.07.2021
(73) Proprietor: Daisankasei Co., Ltd., Tokyo 103-8410 (JP)
(72) Inventor: NAGASHIMA Tadashi, Ichihara-shi, Chiba 290-0045 (JP); INOUE Nao, Tsuruoka-shi, Yamagata 997-8555 (JP)
(74) Representative: Kinkeldey, Daniela
(86) International application number: PCT/JP2019/033822
(87) International publication number: WO 2020/045542

(56) References cited:
- WO-A1-00/69404
- WO-A1-2011/122722
- JP-A- 2001 240 544
- JP-A- 2016 167 991
- KUNIO SUZUKI ET AL: "A Fermentation Product of Phytosterol Including Campestenone Reduces Body Fat Storage and Body Weight Gain in Mice", JOURNAL OF NUTRITIONAL SCIENCE AND VITAMINOLOGY, vol. 53, 1 January 2007 (2007-01-01), pages 63 - 67, XP055690942, ISSN: 0301-4800
- IKEDA I ET AL: "Campest-5-en-3-one, an oxidized derivative of campesterol, activates PPARalpha, promotes energy consumption and reduces visceral fat deposition in rats", BIOCHIMICA ET BIOPHYSICA ACTA, ELSEVIER, AMSTERDAM, NL, vol. 1760, no. 5, 1 May 2006 (2006-05-01), pages 800 - 807, XP025015031, ISSN: 0304-4165, [retrieved on 20060501], DOI: 10.1016/J.BBAGEN.2006.02.017
- KUNIO SUZUKI , RIE KONNO , TAKKESHI SHIMIZU , TADASHI NAGASHIMA , AKIHIKO KIMURA : "A fermentation product of phytosterol including campestenone reduces body fat storage and body weight gain in mice", JOURNAL OF NUTRITIONAL SCIENCE AND VITAMINOLOGY, vol. 53, no. 1, 1 January 2007 (2007-01-01), pages 63 - 67, XP055690942, ISSN: 0301-4800
- IKEDA I; KONNO R; SHIMIZU T; IDE T; TAKAHASHI N; KAWADA T; NAGAO K; INOUE N; YANAGITA T; HAMADA T; MORINAGA Y; TOMOYORI H; IMAIZUM: "Campest-5-en-3-one, an oxidized derivative of campesterol, activates PPARalpha, promotes energy consumption and reduces visceral fat deposition in rats", BIOCHIMICA ET BIOPHYSICA ACTA , vol. 1760, no. 5, 1 May 2006 (2006-05-01), pages 800 - 807, XP025015031, ISSN: 0304-4165, DOI: 10.1016/j.bbagen.2006.02.017
- SUZUKI KUNIO : "Lipid metabolism control agent (5-campestenone)", JAPANESE JOURNAL OF CLINICAL MEDICINE, vol. 61, no. S6, 30 November 2002 (2002-11-30), pages 698 - 703, XP009526165, ISSN: 0047-1852
- JEFFREY J BERTI , JAMES J LIPSKY: "Transcutaneous drug delivery: a practical review", MAYO CLINIC PROCEEDINGS, vol. 70, no. 6, 1 June 1995 (1995-06-01), pages 581 - 586, XP009526177, ISSN: 0025-6196, DOI: 10.4065/70.6.581
- CAROLE RIBET; EMILIE MONTASTIER; CARINE VALLE; VÃÂ©RONIC BEZAIRE; ANNE MAZZUCOTELLI; ALINE MAIRAL; NATHALIE VIGUERIE; DOMINIQUE L: "Peroxisome proliferator- activated receptor-alpha control of lipid and glucose metabolism in human white adipocytes", ENDOCRINOLOGY, vol. 151, no. 1, 1 January 2010 (2010-01-01), pages 123 - 133, XP055690944, ISSN: 0013-7227, DOI: 10.1210/en.2009-0726

## Description

### Technical Field

The present invention relates to a novel use of phytostenone.

### Background Art

It is said that excessive accumulation of fat occurs due to various causes such as overnutrition, insufficient consumption of energy due to lack of exercise, etc., stress or modulation of hormone balance, and after effects of disease. The sites in which fat is accumulated are organs such as liver, mesenteric tissues, subcutaneous tissues, etc. In particular, subcutaneous tissues account for a large area as a fat accumulation site. Among others, a large amount of fat is easily accumulated in the upper arm, the abdomen, and a site ranging from the waist to the femur, and thus, even from a cosmetic perspective, such excessive fat accumulation is problematic.

In order to alleviate excessive accumulation of subcutaneous fat, a large number of attempts have been made, and not only the medical treatment or prevention of the excessive accumulation of subcutaneous fat, but also prevention through dietary intake on a daily basis or various types of beverages has been performed. For example, food materials, such as L-carnitine or a domestic animal meat peptide comprising the same, algae extract, carotenoid, anthocyanins, and green tea catechin, have been proposed as food materials for regulating the metabolism of egg lipids ingested, promoting body fat combustion, or suppressing accumulation of body fat.

However, under the current circumstances, there are only a few materials capable of effectively preventing and improving obesity symptoms, and almost no sufficiently satisfactory materials can be found. Therefore, it has been desired to develop a material that inhibits absorption of fat during a meal, suppresses accumulation of fat in a body, or promotes body fat combustion or the metabolism of body fat.

It has been reported that plant stenone (phytostenone) that is a plant sterol (phytosterol) fermentation product reduces visceral fat and subcutaneous fat, when it is added to a high fat diet, followed by feeding (for example, Non Patent Literatures 1 to 3). It has been considered that the effect of phytostenone to reduce visceral fat and subcutaneous fat by oral administration is provided by various mechanisms of action including acceleration of fat metabolism by β oxidation, inhibition of fatty acid synthesis, and excretion of lipids from the body (for example, Non Patent Literature 1 and Non Patent Literatures 2). However, it has not been known that application of phytostenone onto the skin of a subject can reduce the subcutaneous fat of the subject.

Herein, the use of phytostenone as an additive to food and beverage, a medicament, or the like has been proposed (for example, Patent Literatures 1 to 3). In addition, a method for producing a 5-en-3-one derivative of sterol (i.e., one type of phytostenone) with a high yield has been proposed (for example, Patent Literature 4).

### Citation List

### Patent Literature

Patent Literature 1: JP Patent Publication (Kokai) No. 2001-240544 A
Patent Literature 2: JP Patent Publication (Kokai) No. 2003-48837 A
Patent Literature 3: JP Patent Publication (Kokai) No. 2011-51939 A
Patent Literature 4: JP Patent Publication (Kokai) No. 2007-284348 A

### Non Patent Literature

Non Patent Literature 1: Kunio SUZUKI, et al., Nippon Rinsho (2003), 61, Suppl.6, pp. 698-703
Non Patent Literature 2: Kunio Suzuki, et al., J. Nutr. Sci. Vitaminol. (2007), 53, pp. 63-67
Non Patent Literature 3: Journal of Japan Society for the Study of Obesity (2007), 13(3), pp. 244-249

### Summary of Invention

### Technical Problem

Under the aforementioned circumstances, it has been desire to develop a further method for reducing subcutaneous fat.

### Solution to Problem

The present inventors have conducted intensive studies, and as a result, the present inventors have found that, for example, the subcutaneous fat of a subject can be reduced by applying a phytostenone to the skin of the subject, etc., thereby completing the present invention.

### Advantageous Effects of Invention

According to the present invention, a transdermal absorbent product comprising a phytostenone for therapeutic use and for a non-therapeutic use in reducing subcutaneous fat is provided.

By applying a transdermal absorbent of a preferred aspect onto the skin of a subject, the subcutaneous fat of the subject can be reduced. By applying a transdermal absorbent of another preferred aspect onto the skin of a subject, fat excessively accumulated in the subcutis of the subject can be metabolized and reduced. A transdermal absorbent of a further preferred aspect can reduce fat, which is excessively accumulated in the subcutis of a subject, locally or in a wide range, and which is problematic from a health or beauty perspective, and thus, it can improve the health or beauty of the subject.

### Brief Description of Drawings

[Figure 1] Figure 1 is a view showing the effects of a phytostenone on subcutaneous fat. E: ethanol solution (control), C: campest-5-en-3-one, and P: phytostenone-containing composition.
[Figure 2] Figure 2 is a view showing the effects of a phytostenone on mesenteric fat. E: ethanol solution (control), C: campest-5-en-3-one, and P: phytostenone-containing composition.
[Figure 3] Figure 3 is a view showing the effects of a phytostenone on epididymal fat. E: ethanol solution (control), C: campest-5-en-3-one, and P: phytostenone-containing composition.
[Figure 4] Figure 4 is a view showing the effects of a phytostenone on perirenal fat. E: ethanol solution (control), C: campest-5-en-3-one, and P: phytostenone-containing composition.
[Figure 5] Figure 5 is a view showing the effects of a phytostenone on the liver. E: ethanol solution (control), C: campest-5-en-3-one, and P: phytostenone-containing composition.

### Description of Embodiments

Hereinafter, the present invention will be described in detail.

In several aspects, a transdermal absorbent for reducing subcutaneous fat, wherein the transdermal absorbent comprises a phytostenone, as described in the appended claims, is provided. The phytostenone may be used alone as a single type, or may also be used in combination of two or more types.

The "phytostenone" is an enone compound having a structure in which the hydroxyl group on the carbon at position 3 of phytosterol is substituted with an oxo group, namely, having a carbonyl group at position 3. In the present description, the "phytostenone" may be referred to as a "plant stenone" or a "3-one derivative of phytosterol," and also, the "phytosterol" may be referred to as a "plant sterol."

In the present description, the term "phytostenone" is used to include a phytostenone and an analog thereof. In addition, in the present description, the term "phytosterol" is used to include phytosterol and an analog thereof.

The structure of a phytostenone is not particularly limited, as long as it has a carbonyl group at position 3 of a sterol skeleton. The phytostenone may have other substituents. The phytostenone may have one or more carbonyl groups at a position(s) other than the position 3 of the sterol skeleton. An example of the phytostenone having a carbonyl group(s) at a position(s) other than the position 3 of the sterol skeleton may be a 3,6-dione derivative of phytosterol (having carbonyl groups at positions 3 and 6 of the sterol skeleton). Moreover, the phytostenone may have a functional group, such as an alkyl group (e.g., a methyl group and an ethyl group) and a hydroxyl group, in the sterol skeleton or a side chain thereof. Furthermore, the phytostenone may have a double bond in the structure thereof. The number of such double bonds is not particularly limited, and the phytostenone may have one or more, preferably 1 to 4, and more preferably 1 or 2 other double bonds in the sterol skeleton or a side chain thereof.

Examples of the phytostenone may include a 5-en-3-one derivative of phytosterol, a 4-en-3-one derivative of phytosterol, and a 4-en-3,6-dione derivative of phytosterol.

Examples of the 5-en-3-one derivative of phytosterol may include campest-5-en-3-one, sitost-5-en-3-one, stigmast-5-en-3-one, brassicast-5-en-3-one, 24-alkylcholest-5-en-3-one, 24-alkylcholest-5,7-dien-3-one, 24-alkylcholest-5,8-dien-3-one, 24-alkylcholest-5,9(11)-dien-3-one, 24-alkylcholest-5,22-dien-3-one, 24-alkylcholest-5,7,22-trien-3-one, 24-alkylcholest-5,8,22-trien-3-one, 24-alkylcholest-5,9(11),22-trien-3-one, and 24-alkylcholest-5,25(27)-dien-3-one.

Examples of the 4-en-3-one derivative of phytosterol may include campest-4-en-3-one, sitost-4-en-3-one, stigmast-4-en-3-one, and brassicast-4-en-3-one.

Examples of the 4-en-3,6-dione derivative of phytosterol may include campest-4-en-3,6-dione, sitost-4-en-3,6-dione, stigmast-4-en-3,6-dione, brassicast-4-en-3,6-dione, 6-hydroxy-sitost-4-en-3,6-dione, 4-cholesten-3,6-dione, campest-4-en-3,6-dione, and 24-alkylcholest-4-en-3,6-dione.

Besides, examples of the "alkyl" in each compound may include a methyl group and an ethyl group.

As a phytostenone used herein, a commercially available phytostenone may be used, or a phytostenone produced according to a known chemical or biological method or a method equivalent thereto may also be used. The biological method is a method utilizing enzyme, fermentation, etc. An example of the commercially available phytostenone may be Stigma-4-en-3-one manufactured by Sigma-Aldrich.

In several aspects, a phytostenone-containing composition is used as a phytostenone. Such a phytostenone-containing composition can be obtained, for example, by culturing microorganisms in a medium comprising phytosterol or a plant body containing the phytosterol to conduct fermentation. Such a method is described, for example, in JP Patent Publication (Kokai) No. 2007-284348 A, and J. Nutr. Sci. Vitaminol 53, 63-67. 2006. In several aspects, the phytostenone-containing composition comprises a 5-en-3-one derivative of phytosterol, a 4-en-3-one derivative of phytosterol, and a 4-en-3,6-dione derivative of phytosterol. In several aspects, the phytostenone-containing composition comprises the 5-en-3-one derivative of phytosterol, the 4-en-3-one derivative of phytosterol, and the 4-en-3,6-dione derivative of phytosterol, in an amount of 40% by weight or more (for example, 40% by weight or more, 41% by weight or more, 42% by weight or more, 43% by weight or more, 44% by weight or more, 45% by weight or more, 46% by weight or more, 47% by weight or more, 48% by weight or more, 49% by weight or more, 50% by weight or more, 51% by weight or more, 52% by weight or more, 53% by weight or more, 54% by weight or more, 55% by weight or more, 56% by weight or more, 57% by weight or more, 58% by weight or more, 59% by weight or more, and 60% by weight or more). In several preferred aspects, the phytostenone-containing composition comprises: a 5-en-3-one derivative of at least one phytosterol selected from the group consisting of campest-5-en-3-one, sitost-5-en-3-one, stigmast-5-en-3-one, brassicast-5-en-3-one, and a combination thereof; a 4-en-3-one derivative of phytosterol selected from the group consisting of campest-4-en-3-one, sitost-4-en-3-one, stigmast-4-en-3-one, brassicast-4-en-3-one, and a combination thereof; and a 4-en-3,6-dione derivative of phytosterol selected from the group consisting of campest-4-en-3,6-dione, sitost-4-en-3,6-dione, stigmast-4-en-3,6-dione, brassicast-4-en-3,6-dione, and a combination thereof. In several other preferred aspects, the phytostenone-containing composition comprises sitost-5-en-3-one, sitost-4-en-3-one, campest-5-en-3-one, campest-4-en-3-one, and phytost-4-en-3,6-dione (namely, campest-4-en-3,6-dione, sitost-4-en-3,6-dione, stigmast-4-en-3,6-dione, and brassicast-4-en-3,6-dione).

Herein, components other than the phytostenone comprised in the phytostenone-containing composition may be, for example, plant sterols serving as substrates (namely, sitosterol, campesterol, stigmasterol, and brassicasterol).

Examples of the phytosterol may include campesterol, β-sitosterol, stigmasterol, brassicasterol, and 24-alkylcholesterol. As phytosterol, a commercially available product may be obtained and used, or phytosterol may be produced according to a known method or a method equivalent thereto.

The "plant body containing phytosterol" may be a plant body as a whole containing phytosterol, or may also be a part of such a plant body (e.g., a stem, a leaf, a root, a flower, a fruit, and a seed). Examples of the plant body containing phytosterol may include all plants, such as, for example, soybean, sunflower, rapeseed, sea buckthorn, and pine.

The microorganisms used in the culture may be, for example, microorganisms exhibiting cholesterol oxidase activity. Examples of the microorganisms may include Basidiomycetes (e.g., genus Agaricus and genus Trametes), Filamentous fungi (e.g., genus Aspergillus and genus Monascus), and bacteria (e.g., genus Arthrobacter, genus Streptomyces, genus Brevibacteriumu, genus Rhodococcus, and genus Bacillus). In several aspects, a specific example of the microorganisms may be *Arthrobacter Simplex* (alias: *Nocardioidos Simplex*)*.*

As such microorganisms, commercially available microorganisms may be used, or microorganisms with mutated mycological properties obtained by artificial mutation means (for example, chemical substances (e.g., EMS (ethyl methanesulfonate), diethyl sulfate, NTG (N-methyl-N'-nitro-N-nitrosoguanidine) and nitrosoguanidine), X-ray, γ-ray, and ultraviolet ray) may also be used. Alternatively, a gene encoding choresterol oxidase is introduced into microorganisms (e.g., *Escherichia coli*) using a suitable vector, to transform the microorganisms, and so, the transformed microorganisms capable of the expression of the choresterol oxidase may also be used.

The amount of the phytostenone mixed into the transdermal absorbent can be set, as appropriate. The amount of the phytostenone mixed is, for example, 0.1% to 20% by weight, and preferably 0.5% to 10% by weight.

Into the transdermal absorbent, not only the phytostenone, but also the following components used in medicaments or cosmetics can be mixed, as necessary. Examples of the components other than the phytostenone that are mixed into the transdermal absorbent may include alcohols (e.g., ethanol, isostearyl alcohol, cetyl alcohol, phenoxy ethanol, etc.), emulsifiers (e.g., polyglycerin fatty acid ester, a lecithin derivative, polyethylene glycol fatty acid ester, sorbitan fatty acid ester, and a polymeric emulsifier), transdermal absorption promoters (e.g., fatty acid, aliphatic alcohol, fatty acid ester, alkyl ether, aromatic organic acid, aromatic alcohol, aromatic organic acid ester, aryl ether, and a ribosome preparation), water (e.g., purified water, hot spring water, and deep water), oil agents, surfactants, metal soaps, gelling agents, powders, water-soluble polymers, film-forming agents, resins, UV protection agents, inclusion compounds, antibacterial agents, flavoring agents, deodorants, salts, pH adjusters, refreshing agents, animal/microorganism-derived extracts, plant extracts, blood circulation promoters, astringents, anti-seborrheic agents, whitening agents, anti-inflammatory agents, active oxygen scavengers, cell activators, moisturizers, chelating agents, keratolytic agents, enzymes, hormones, and vitamins. These components may be used alone as a single type, or may also be used in combination of two or more types, as appropriate, depending on the dosage form of the transdermal absorbent. For example, since the phytostenone is insoluble in water, it may be dissolved in alcohols (e.g., ethanol) so that it may be converted to a liquid state, or it may be converted to a cream state, using an emulsifier. In several aspects, the transdermal absorbent comprises a transdermal absorption promoter.

In several aspects, the transdermal absorbent is a medicament for reducing subcutaneous fat. The medicament is not particularly limited, as long as it can be applied to the skin. The medicament may have a liquid state or a semi-solid state (e.g., gel, paste, and cream). Examples of the medicament may include a gelling agent, a cream agent, a liquid agent, a patch, an aerosol agent, an aqueous ointment, and a pack.

In several other aspects, the transdermal absorbent is a cosmetic for reducing subcutaneous fat. The cosmetic is not particularly limited, as long as it can be applied to the skin. The cosmetic may have a liquid state or a semi-solid state (e.g., gel, paste, and cream). Examples of the cosmetic may include basic cosmetics (e.g., skin toner, cream, milky lotion, and essence), hair cosmetics (shampoo, conditioner, and treatment), make up cosmetics (e.g., liquid foundation and base emulsion), and sunscreen cosmetics.

In several aspects, the transdermal absorbent is used to apply onto the skin of a subject, in order to reduce subcutaneous fat in the subject in need of a reduction of the subcutaneous fat.

In addition, provided herein is a method for reducing the subcutaneous fat of a subject, comprising applying phytostenone onto the skin of a subject in need of a reduction of subcutaneous fat. Otherwise, a phytostenone for reducing subcutaneous fat, which is used to apply onto the skin, is provided.

The "subject" is a human, or an organism other than the human, such as, for example, birds and non-human mammals (e.g., a bovine, a monkey, a cat, a mouse, a rat, a guinea pig, a hamster, a swine, a dog, a rabbit, sheep, and a horse). The "subject" is preferably a human.

The term "to apply" is used to mean that the phytostenone is applied onto the skin of a subject in an amount sufficient for reducing the subcutaneous fat of the subject. In several aspects, the phytostenone is applied onto the skin that is present on subcutaneous tissues, in which subcutaneous fat needed to be reduced in the subject is accumulated. Preferably, the phytostenone is applied to the site of a subject, in which subcutaneous fat is easily accumulated, for example, to the skin on a site selected from the upper arm, the abdomen, and a site ranging from the waist to the femur. The transdermal absorbent or the phytostenone can be applied onto the skin by coating, adhesion, spraying, etc., depending on the dosage form thereof.

The applied dose of the phytostenone is different depending on age, sex, symptoms, the number of doses, dosage form, etc. With regard to the applied dose, the phytostenone may be administered at a number of doses that is from once to several times a day, and for example, at a dose of 5 mg/day to 1000 mg/day, or 50 mg/day to 500 mg/day.

In several preferred aspects, the transdermal absorbent, the method, or the phytostenone can reduce subcutaneous fat, more preferentially than visceral fat.

In several aspects, the transdermal absorbent, the method, or the phytostenone may be used in combination with oral administration of the phytostenone.

In several aspects, the transdermal absorbent, the method, or the phytostenone may be used in combination with other pharmaceutical active ingredients or other cosmetic active ingredients.

Thepresent description refers to the claims, specification and/or drawings of Japanese Patent Application No. 2018-161943 (filed on August 30, 2018), which is a priority document of the present application.

In addition, the purposes, characteristics, advantages, and ideas of the present invention are apparent to a person skilled in the art by the descriptions of the present description, and such a skilled person could readily carry out the present invention based on the descriptions of the present description. The modes for carrying out the invention, specific examples, and the like are provided to show the preferred embodiments of the present invention, and are provided for illustration or explanation only, and thus, these examples are not intended to limit the scope of the present invention. It is clear to a person skilled in the art that various modifications can be made to the present invention based on the descriptions of the present description within the intention and range of the present invention disclosed in the present description.

### Examples

### Example 1: Method for preparing plant stenone that is subcutaneous fat metabolism promoter

A phytostenone-containing composition used as a test substance was produced according to a method equivalent to the fermentation method described in JP Patent Publication (Kokai) No. 2007-284348 A. Specifically, gram-positive bacteria had previously been cultured in a medium containing phytosterol to prepare a culture solution. To the prepared culture solution, phytosterol was added, and an organic solvent was further layered thereon, followed by the intensive stirring of the obtained mixture with an impeller. Thereafter, an upper layer was recovered by two-layer separation, and was then dried and solidified to obtain a phytostenone-containing composition. The obtained phytostenone-containing composition comprised 50% by weight or more of various types of stenones.

Sitost-5-en-3-one, sitost-4-en-3-one, campest-5-en-3-one, campest-4-en-3-one, and phytost-4-en-3,6-dione.

Herein, the "phytost-4-en-3,6-dione" is a mixture of campest-4-en-3,6-dione, sitost-4-en-3,6-dione, stigmast-4-en-3,6-dione, and brassicast-4-en-3,6-dione.

Various types of phytostenones and phytosterols were analyzed by high performance liquid chromatography according to the method described in JP Patent Publication (Kokai) No. 2007-284348 A. The analyzed numerical values are as follows.

5-en-3-one derivative (12.5% by weight), 4-en-3-one derivative (36.6% by weight), and 4-en-3,6-dione derivative (2.8% by weight). (Total: 51.9% by weight)

It is to be noted that remaining components in the phytostenone-containing composition are plant sterols serving as substrates (namely, sitosterol, campesterol, stigmasterol, and brassicasterol), etc.

Campest-5-en-3-one used as a test substance was synthesized by the selective oxidation method of Parish et al. or the Swern oxidation method, using Campesterol (TAMA BIOCHEMICAL CO., LTD.) as a raw material (Parish E.J., et al: Synthetic communications (1992) 22, pp. 2839-).

SD rats (Oriental Yeast Co., Ltd.), which had previously been fed with a standard purified sample AIN-93G (Oriental Yeast Co., Ltd.) for 2 weeks and thus had been fattened, were used. The abdomen of each fat rat was dehaired (20 mm square). Thereafter, 2 ml of a phytostenone-containing composition (30 mg/ml ethanol solution) used as a test substance, 2 ml of campest-5-en-3-one (5 mg/ml ethanol solution) used as a test substance, or 2ml of an ethanol solution used as a control was applied gently with a cotton to individual rats divided into the groups (n = 3 in each group). This operation was carried out continuously for 2 weeks. After termination of the application test, the rats were dissected, and liver fat, epididymal fat, perirenal fat, mesenteric fat, and subcutaneous fat were carefully recovered. Thereafter, the weight of each fat was measured.

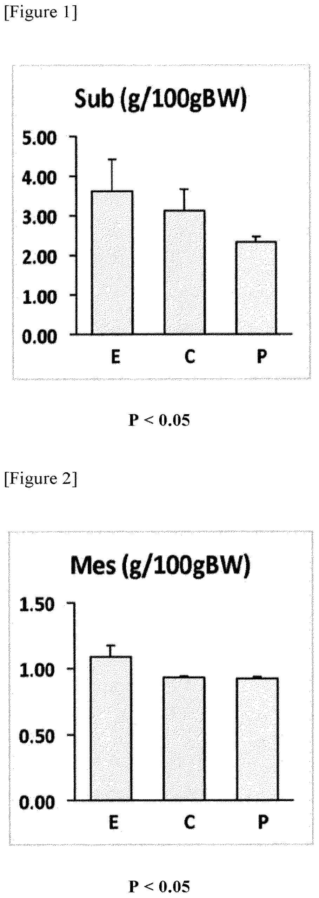

As a result, the amount of subcutaneous fat was significantly reduced (p < 0.05) in the case of applying the phytostenone-containing composition (P), in compared with the case of applying the control (E), and approximately 40% of the subcutaneous fat was reduced (Figure 1). Moreover, the amount of subcutaneous fat was significantly reduced (p < 0.05) also in the case of applying the campest-5-en-3-one (C), in compared with the case of applying the control (E), and approximately 16% of the subcutaneous fat was reduced.

In view of the foregoing, the subcutaneous fat after application of the phytostenone-containing composition and the subcutaneous fat after application of the campest-5-en-3-one were reduced by 40% and 16%, respectively, with respect to the case of application of the control. The dose of phytostenone applied by transdermal administration of the phytostenone-containing composition was approximately 31 mg per day (30 mg/ml x 2 ml x 0.519), and thus, the total dose of phytostenone for 2 weeks was approximately 434 mg. Moreover, the dose of phytostenone applied by transdermal administration of the campest-5-en-3-one was approximately 10 mg per day (5 mg/ml x 2 ml), and thus, the total dose of phytostenone for 2 weeks was only approximately 140 mg.

On the other hand, it has been reported that when the campest-5-en-3-one that strongly reduces visceral fat was orally administered at a dose of approximately 120 mg/day over 8 weeks, the subcutaneous fat reduction percentage was found to be 26% (Journal of Japan Society for the Study of Obesity (2007), 13(3), pp. 244-249). Since the dose of the campest-5-en-3-one by oral administration is approximately 120 mg per day, it would reach approximately 6720 mg for a total of 8 weeks.

From these results, it is found that when phytostenone is transdermally administered, subcutaneous fat is reduced in a shorter time and with a smaller amount of phytostenone, in comparison to the oral administration thereof.

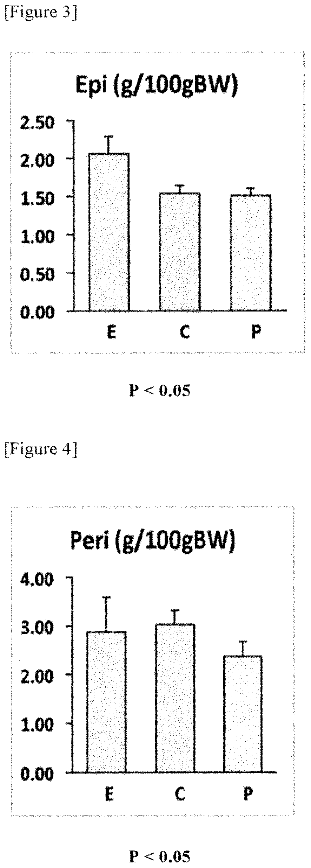

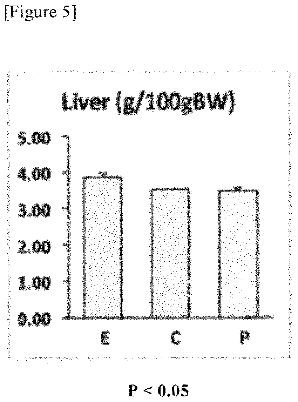

It has been known that, in general, visceral fat is easily reduced but subcutaneous fat is hardly reduced. It has also been known that the metabolism of subcutaneous fat is often carried out after the metabolism of visceral fat has been completed, and that the subcutaneous fat is preferentially metabolized when aerobic exercise has been done. Fats existing in the sites around the mesentery, around the kidney, around the testis, the liver and the like are referred to as visceral fats. However, the amounts of the fats existing in these sites were not significantly reduced by application of the test substance, when compared with the case of application of the control (Figures 2 to 5). That is, it is found that subcutaneous fat was preferentially reduced compared with visceral fat, when the test substance was applied thereto.

Herein, in the present example, the significance test was carried out as follows. First, the mean value ± standard error in each group was calculated, and a variance test was then carried out. In the case of equal variance, the significance test was carried out according to the t-test, and in the case of unequal variance, the significance test was carried out according to the Welch method.

From the present example, it became clear that when phytostenone is transdermally administered, subcutaneous fat is reduced in a shorter time and with a smaller amount of phytostenone, in comparison to the oral administration thereof.

## Claims

1. A transdermal absorbent product for therapeutic use in reducing subcutaneous fat, wherein the transdermal absorbent comprises a phytostenone.

2. The transdermal absorbent for use according to claim 1, wherein the phytostenone is a phytostenone-containing composition.

3. The transdermal absorbent for use according to claim 2, wherein the phytostenone-containing composition comprises a 5-en-3-one derivative of phytosterol, a 4-en-3-one derivative of phytosterol, and a 4-en-3,6-dione derivative of phytosterol.

4. The transdermal absorbent for use according to claim 1, wherein the phytostenone is campest-5-en-3-one.

5. A non-therapeutic method for reducing subcutaneous fat in a subject, which comprises applying a transdermal absorbent comprising a phytostenone onto the skin of a subject in need of a reduction of subcutaneous fat.

6. The non-therapeutic method for reducing subcutaneous fat in a subject according to claim 5, wherein the phytostenone is a phytostenone-containing composition.

7. The non-therapeutic method for reducing subcutaneous fat in a subject according to claim 6, wherein the phytostenone-containing composition comprises a 5-en-3-one derivative of phytosterol, a 4-en-3-one derivative of phytosterol, and a 4-en-3,6-dione derivative of phytosterol.

8. The non-therapeutic method for reducing subcutaneous fat in a subject according to claim 5, wherein the phytostenone is campest-5-en-3-one.

## Patentansprüche

1. Transdermales Absorbensprodukt zur therapeutischen Verwendung beim Verringern von subkutanem Fett, wobei das transdermale Absorbens ein Phytostenon umfasst.

2. Transdermales Absorbens zur Verwendung nach Anspruch 1, wobei es sich bei dem Phytostenon um eine phytostenonhaltige Zusammensetzung handelt.

3. Transdermales Absorbens zur Verwendung nach Anspruch 2, wobei die phytostenonhaltige Zusammensetzung ein 5-En-3-on-Derivat von Phytosterol, ein 4-En-3-on-Derivat von Phytosterol und ein 4-En-3,6-dion-Derivat von Phytosterol umfasst.

4. Transdermales Absorbens zur Verwendung nach Anspruch 1, wobei es sich bei dem Phytostenon um Campest-5-en-3-on handelt.

5. Nichttherapeutisches Verfahren zur Verringerung von subkutanem Fett bei einem Individuum, das ein Auftragen eines ein Phytostenon umfassenden transdermalen Absorbens auf die Haut eines eine Verringerung von subkutanem Fett benötigenden Individuums umfasst.

6. Nichttherapeutisches Verfahren zur Verringerung von subkutanem Fett bei einem Individuum nach Anspruch 5, wobei es sich bei dem Phytostenon um eine phytostenonhaltige Zusammensetzung handelt.

7. Nichttherapeutisches Verfahren zur Verringerung von subkutanem Fett bei einem Individuum nach Anspruch 6, wobei die phytostenonhaltige Zusammensetzung ein 5-En-3-on-Derivat von Phytosterol, ein 4-En-3-on-Derivat von Phytosterol und ein 4-En-3,6-dion-Derivat von Phytosterol umfasst.

8. Nichttherapeutisches Verfahren zur Verringerung von subkutanem Fett bei einem Individuum nach Anspruch 5, wobei es sich bei dem Phytostenon um Campest-5-en-3-on handelt.

## Revendications

1. Produit absorbant transdermique destiné à une utilisation thérapeutique pour réduire la graisse sous-cutanée, l'absorbant transdermique comprenant une phytosténone.

2. Absorbant transdermique destiné à être utilisé selon la revendication 1, la phytosténone étant une composition contenant de la phytosténone.

3. Absorbant transdermique destiné à être utilisé selon la revendication 2, la composition contenant de la phytosténone comprenant un dérivé 5-én-3-one de phytostérol, un dérivé 4-én-3-one de phytostérol et un dérivé 4-ène-3,6-dione de phytostérol.

4. Absorbant transdermique destiné à être utilisé selon la revendication 1, la phytosténone étant une campest-5-én-3-one.

5. Procédé non thérapeutique de réduction de la graisse sous-cutanée chez un sujet, qui comprend l'application d'un absorbant transdermique comprenant une phytosténone sur la peau d'un sujet ayant besoin d'une réduction de la graisse sous-cutanée.

6. Procédé non thérapeutique de réduction de la graisse sous-cutanée chez un sujet selon la revendication 5, dans lequel la phytosténone est une composition contenant de la phytosténone.

7. Procédé non thérapeutique de réduction de la graisse sous-cutanée chez un sujet selon la revendication 6, dans lequel la composition contenant de la phytosténone comprend un dérivé 5-én-3-one de phytostérol, un dérivé 4-én-3-one de phytostérol et un dérivé 4-ène-3,6-dione de phytostérol.

8. Procédé non thérapeutique de réduction de la graisse sous-cutanée chez un sujet selon la revendication 5, dans lequel la phytosténone est la campest-5-én-3-one.
